# EUROPEAN PATENT APPLICATION

(11) **EP 1 452 166 A1**
(43) Date of publication of application: **01.09.2004**
(21) Application number: 02801526.1
(22) Date of filing: 10.10.2002
(51) Int. Cl.: A61K 7/06, A61K 33/24, A61P 17/16, C02F 1/68, C02F 1/46

(54) **HAIR REPAIRING LIQUID COMPRISING WATER DISPERSED WITH ULTRAFINE PARTICLE TITANIUM GROUP METAL BY PLASMA UNDERWATER DISCHARGE AND METHOD AND SYSTEM FOR PRODUCING THE SAME**

(30) Priority: 12.10.2001 JP 2001315337
(71) Applicant: Phild Co., Ltd., Kyoto city, Kyoto pref. 604-8152 (JP)
(72) Inventor: HIRATA, Y. Phild Co., Ltd. 678, Tearaimizu-cho, Kyoto city, Kyoto pref. 604-8152 (JP); UEDA, Y. Phild Co., Ltd. 678, Tearaimizu-cho, Kyoto city, Kyoto pref. 604-8152 (JP); TAKASE,H. Phild Co., Ltd. 678, Tearaimizu-cho, Kyoto city, Kyoto pref. 604-8152 (JP); SUZUKI, K. Phild Co., Ltd. 678, Tearaimizu-cho, Kyoto city, Kyoto pref. 604-8152 (JP)
(74) Representative: VOSSIUS & PARTNER
(86) International application number: PCT/JP2002/010523
(87) International publication number: WO 2003/032932

(57) **Abstract**

Provide a micro-dispersion water of super-fine titanium-group metal grains offering hair-repairing effects, as well as its production method and apparatus.

Efficiently produce a concentration-adjustable water solution of super-fine titanium-group metal grains using a highly safe, compact production apparatus that comprises a power supply for high-voltage/current discharge, a titanium-group metal electrode feeder, an electrode-vibrating/sliding device, a high-voltage discharge generator equipped with a titanium-group metal electrode and a counter electrode, a water tank, a water inlet to the water tank, and an outlet for generated water solution of super-fine titanium-group grains, wherein plasma discharge is caused between the titanium-group metal electrode and counter electrode in water to let ion vapor of titanium-group metal contact and dissolve in water.

## Description

### Field of the Invention

This invention relates to a hair-repair liquid comprising high-function water in which super-fine grains of titanium-group metal are micro-dispersed.

The invention also relates to a method and apparatus for producing a hair-repair liquid comprising high-function water in which super-fine grains of titanium-group metal are micro-dispersed.

### Background of the Invention

Titanium-group metals including titanium, zirconium, hafnium and thorium are relatively new metal materials discovered much more recently than iron, copper and aluminum. The lightweight metals offering excellent strength at high temperature are already used in wide industrial applications, such as jet engine material for aircraft and spaceship, tube and tube plate comprising heat-exchangers used for nuclear or thermal power generation, and articles of daily use such as eyeglass frame and golf club head. The applications of titanium-group metals are expected to grow further.

A relatively large number of examples have been known to date regarding the use of titanium metal in articles of daily use, health/medical products and cosmetics. These examples include barber's scissors with titanium film coatings (Japanese Patent Application Laid-open No. 62-268584), the utilization of far-infrared ray through molten titanium metal (Japanese Patent Application Laid-open Nos. 61-59147, 1-155803 and 3-112849), bedding (Japanese Patent Application Laid-open No. 8-322695), cooking utensil (Japanese Patent Application Laid-open No. 9-140593), eye mask (Japanese Patent Application Laid-open No. 10-71168), health-maintaining equipment (Japanese Patent Application Laid-open Nos. 11-285541 and 11-285543), health band (Registered Japanese Utility Model No. 3045835) and health slippers (Registered Japanese Utility Model No. 3061466).

More than a few applications of titanium compounds for hair treatment purposes have been proposed (the examples in this field include Japanese Patent Application Laid-open Nos. 57-18606, 1-113313, 1-224310, 4-124121 and 5-170635, Japanese Patent Application Nos. 4-511289 and 5-504989, Japanese Patent Application Laid-open Nos. 11-315006 and 60-67210, Publication of Examined Patent Application No. 5-45565, Japanese Patent No. 1850423, Japanese Patent Application Laid-open No. 63-270620, Japanese Patent No. 2567861, Japanese Patent Application Laid-open No. 1-254617, Japanese Patent No. 2855205, Japanese Patent Application Laid-open No. 3-141216, Japanese Patent No. 2837196, Japanese Patent Application Laid-open Nos. 5-43431, 10-291927, 11-35434, 11-49650, 11-139946 and 11-292744). However, the example of producing water that contains titanium is limited to one that uses titanium metal in the cathode to produce drinking water through electroosmosis (Japanese Patent Application Laid-open No. 50-40779).

### Summary of the Invention

Use of titanium-group metals such as titanium in bioactive materials, food materials and medical products is generating keen interests. However, no practical technologies have been developed to date that embody such applications.

The present invention aims to apply titanium-group metals, which are less workable than other metals but nonetheless offer almost infinite utilities, as bioactive materials and in health/medical products in particular.

The purpose of the present invention is to provide a hair-repair liquid comprising high-function water in which super-fine grains of titanium-group metal are micro-dispersed, wherein such super-fine titanium-group metal grains are produced from collision of water molecules and the titanium-group metal ions generated by underwater plasma discharging of titanium-group metal in a water tank, and also to provide a method and apparatus for producing the same.

In the context of the present invention, "titanium-group metal micro-dispersion water" refers to water in which super-fine titanium-group metal grains of micron to nanoscale are micro-dispersed.

The basic feature of the present invention lies in the formation of a hair-repair liquid comprising high-function water in which super-fine titanium-group metal grains are micro-dispersed, by way of plasma discharging of titanium-group metal in water. Specifically, the present invention comprises (1) through (3) below:
1. A hair-repair liquid comprising a micro-dispersion water of titanium-group element in which super-fine titanium-group metal grains are micro-dispersed.
2. A method for producing a hair-repair liquid in which super-fine titanium-group metal grains are micro-dispersed, wherein a high-voltage discharge generator equipped with a titanium-group metal electrode and a counter electrode is used to cause plasma discharge between the electrodes in water, thereby allowing super-fine titanium-group metal grains to micro-disperse in water.
3. An apparatus for producing a hair-repair liquid in which super-fine titanium-group metal grains are micro-dispersed, comprising a power supply for high-voltage/current discharge, a high-voltage discharge generator equipped with a titanium-group metal electrode and a counter electrode, a water tank, a water inlet to the water tank, an outlet for generated water solution of super-fine titanium-group metal grains, and a discharge pump.

Generally in a micro-dispersion solution or mixed solution in which super-fine grains of titanium-group metals or oxides of these metals are simply micro-dispersed, the titanium-group metal settles and separates within a short period of time. The most significant feature of the titanium-group metal micro-dispersion water obtained by the present invention is that ionized titanium-group metal that is generated by plasma discharge in water and thereby micro-dispersed in water remain as micro-dispersed grains for a long period of time without settling or otherwise separating themselves from water. The micro-dispersion water of titanium-group metal obtained by the present invention therefore exhibits the surprising, remarkable effect of providing a bioactive hair-repair liquid through an interaction of water molecules and super-fine titanium-group metal grains.

Of the various applications of the water obtained by the present invention, its use as a hair-repair liquid is explained. In general, hair is damaged by external irritations such as friction, heat from the sun, perm chemicals and inappropriate cuts, as well as by malnutrition caused by an unbalanced diet or drastic weight loss. Since damaged hair has no self-repairing ability, it must be protected externally to prevent further damage.

Treatment products are used for this purpose. As is the case with the skin, water and oil are the most important constituents of the hair. In particular, water is absorbed into the hair cortex, which is a component of hair, and adds moisture and a supple look to the hair while making the hair more flexible so as to let it bounce as the body moves. Oil forms an oil film over the hair cuticle and adds shine and gloss to the hair while minimizing friction and preventing damage to the hair cuticle. The high-function titanium-group metal micro-dispersion water obtained by the present invention has been confirmed to provide excellent treatment effects not achieved by other commercial treatment products available today, with the water exhibiting a significant repair effect on damaged hair.

In addition to hair-repair liquids, the high-function titanium-group metal micro-dispersion water obtained by the present invention can also be used in health products such as motor-function enhancing creams, medical products such as antibacterial agents, UV-protection products, healthy drinking water, health supplements, cosmetics, food preservatives, freshness-keeping agents for food, insect repellents, deodorants, and so on.

The titanium-group metal micro-dispersion water obtained by the present invention can be used suitably for the above purposes today, but it is believed that this micro-dispersion water will provide an innovative bioactive material that would fully answer the needs of today's health-conscious consumers.

The mechanism as to why a micro-dispersion water of super-fine titanium-group metal grains obtained by the present invention provides excellent bioactive functions is not yet clear. The inventors are working diligently to find scientific explanations for these functions.

### Brief Description of the Drawings

Fig. 1: Production flow chart of a hair-repair liquid made of titanium-group metal micro-dispersion water as proposed by the present invention
Fig. 2: Schematic drawing of an apparatus for producing a hair-repair liquid made of titanium-group metal micro-dispersion water as proposed by the present invention
Fig. 3: Microphotograph showing the condition of hair treated with a hair-repair liquid made of titanium micro-dispersion water as proposed by the present invention
Fig. 4: Microphotograph showing the condition of hair treated with normal water

### Description of the Symbols

1: High-voltage discharge generator
2: Power supply for high-voltage/current discharge
3: Water tank
4: Titanium-group metal electrode feeder
5: Electrode-vibrating/sliding device
6: Titanium-group metal electrode
7: Counter electrode
8: Water inlet
9: Outlet of processed water
10: Discharge pump
11: Filter system
12: Product

### Best Mode for Carrying Out the Invention

A hair-repair liquid made of titanium-group metal micro-dispersion water as obtained by the present invention is a new product not heretofore available. The present invention also provides a new method and apparatus for producing a hair-repair liquid made of water that contains titanium-group metal as super-fine grains, wherein the production of the titanium-group metal micro-dispersion water is based on melting titanium-group metal not through general methods like thermal melting, arc discharge and laser irradiation that are commonly used to melt metal, but by ionizing titanium-group metal via plasma discharge in water and dispersing the resulting super-fine grains in water.

Specifically, after studying ways to efficiently and economically produce a hair-repair liquid made of titanium-group metal micro-dispersion water, as well as ways to apply such micro-dispersion water to bioactivation purposes, the inventors conceptualized a method to insert a bar made of pure titanium-group metal in water and cause plasma discharge from the bar, thereby causing the generated titanium-group metal ions to collide with water molecules and letting super-fine titanium-group metal grains disperse in water, wherein such plasma discharge is caused in a water tank so as not to produce substances other than water and titanium-group metal.

The method proposed by the present invention requires that the feed rate of material titanium-group metal be controlled, in order to add excellent bioactivity to the obtained titanium-group metal micro-dispersion water. In the aforementioned production method, an appropriate filter system is also necessary because not only super-fine titanium-group metal grains but also a trace amount of titanium-oxide grains of larger grain size will be produced in water.

A method for producing a hair-repair liquid made of titanium-group metal micro-dispersion water as obtained by the present invention through the aforementioned method, as well as an apparatus to embody this method, are explained according to the drawings.

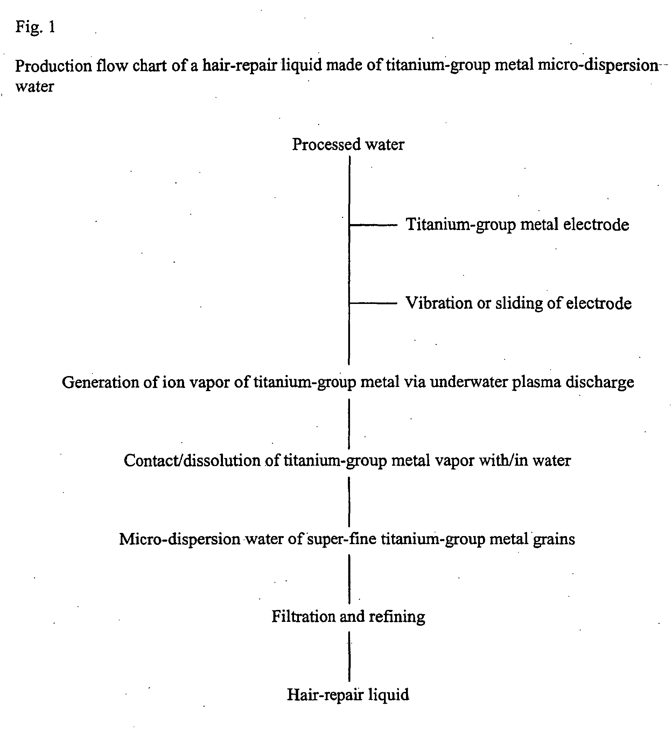

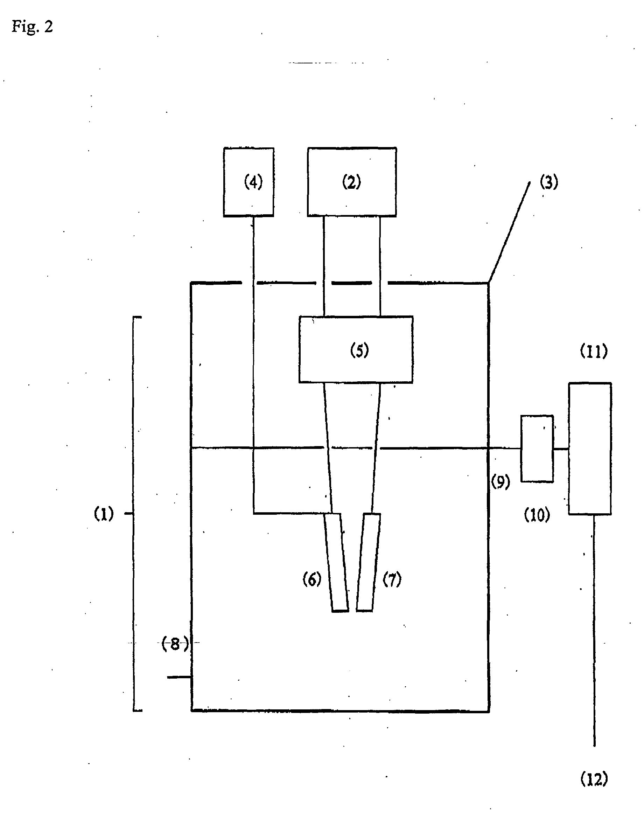

Fig. 1 is a production flow chart for hair-repair liquid made of titanium-group metal micro-dispersion water obtained by the present invention, while Fig. 2 shows an apparatus for producing a hair-repair liquid made of titanium-group metal micro-dispersion water obtained by the present invention.

The apparatus for producing titanium-group metal micro-dispersion water obtained by the present invention, as shown in Fig. 2, comprising a water tank (3) used for producing water in which ionized titanium-group metal is micro-dispersed, a high-voltage discharge generator (1) equipped with an electrode made of titanium-group metal (6) and its counter electrode (7), a feeder of titanium-group metal electrode (4), a device to vibrate or slide the electrode (5), a water inlet (8), an outlet for processed water (9), a discharge pump (10), and a filter system (11) to filter titanium-group metal micro-dispersion water.

The basic structure of the underwater plasma discharge generator proposed by the present invention comprises a production apparatus that comprises a water tank (3), a high-voltage discharge generator (1) equipped with a titanium-group metal electrode and its counter electrode, a water inlet (8), and an outlet (9) through which to discharge titanium-group metal micro-dispersion water. A filter system (11) to filter the produced titanium-group metal micro-dispersion water is also attached as an adjunct.

The water tank proposed by the present invention is made of metal, or preferably steel. The water inlet (8) and outlet (9) are provided on the water tank (3) so the processed water can be retrieved successively.

The titanium-group metal electrode (6) is fed into the water tank from the feeder (4) in accordance with its melting amount. Titanium-group metal ions generated by underwater plasma discharge between the titanium-group metal electrode (6) and its counter electrode (7) make contact with and dissolve in water, thereby becoming super-fine grains. The processed water containing these super-fine titanium-group metal grains is retrieved from the outlet (9) provided at the bottom of the water tank and filtered sequentially through the appropriate filter system (11).

In this apparatus, metal ion vapor is generated from the titanium-group metal electrode by causing plasma discharge in water between the electrode and its counter electrode, and this metal ion vapor is caused to contact water to produce a water dispersant in which super-fine grains of titanium-group metal are micro-dispersed. The titanium-group metal electrode is inserted at a constant rate from the electrode feeder (4) in accordance with the amount of generated metal ion vapor.

In the water tank (3), ionized titanium-group metal (12) is released into water from the electrode and caused to collide with water molecules to produce super-fine grains. At this time, a part of titanium-group metal is considered to take on a crystalline structure.

Thus generated super-fine grains of titanium-group metal, which has a very strong hydrophobic property, remain stably micro-dispersed in water. They remain dispersed for a long period and will not settle even when a coagulating agent is added.

In this apparatus, the electrode made of pure titanium metal (6) is sequentially inserted into the tank containing processing water (3) in a manner vibrating or sliding the electrode.

This apparatus requires plasma discharge to be caused in water, in order to prevent production of substances other than super-fine grains of the material titanium-group metal. This way, vapor of pure titanium-group metal ions free from impurities can be generated in the process of micro-dispersing super-fine titanium-group metal grains in water.

Other characteristics of the present invention include the use of thus produced titanium-group metal micro-dispersion water, in which super-fine titanium-group metal grains are micro-dispersed, as a material for hair-repair liquids and other health products, cosmetics, food materials, drugs and quasi-drugs after necessary refining. The produced titanium-group metal micro-dispersion water contains a small amount of fine titanium-oxide grains of larger grain size generated via bonding of titanium and oxygen and must therefore be filtered and refined as necessary.

So as not to remove the produced super-fine titanium-group metal grains more than necessary, filtering should preferably be implemented not by using ion exchange or reverse-osmosis membrane, but by using the filter system as explained below that can yield a titanium-group metal micro-dispersion water suitable for a desired purpose. For example, filtering the high-pressure water discharged from the high-pressure water tank sequentially through hollow-fiber membranes is beneficial in terms of enhancing the characteristics of titanium micro-dispersion water and extending the filter life, and such filter system will provide a titanium-group metal micro-dispersion water that can meet the required standards governing food and sanitation, cosmetics and drugs.

The constituent analysis of a titanium-group metal micro-dispersion water as obtained by the present invention found a trace amount of super-fine titanium-group metal grains and a very small amount of metal in the water.

### Example:

An embodiment of the present invention is explained in details using an example. Note, however, that the present invention is not limited to this example.

Fig. 2 shows the production apparatus proposed by the present invention.

The production apparatus comprises a water tank (3), a high-voltage discharge generator (1) equipped with a titanium electrode (6) and a counter electrode made of titanium or carbon (7), a water inlet (8), an outlet for processed water (9), a discharge pump (10), and an electrode-vibrating/sliding device (5).

Titanium-group metal ions generated in the water tank (3) via underwater plasma discharge are caused to collide with water molecules to obtain a micro-dispersion water in which super-fine titanium grains are micro-dispersed.

As explained above, water is fed into the water tank and electrical current is charged to the high-voltage discharge generator equipped with an electrode made of titanium-group metal and a counter electrode, thereby generating ionized vapor of titanium-group metal via underwater plasma discharge and causing the ionized vapor to contact and dissolve in water to produce super-fine grains. When titanium-group metal is fed at a rate of 0.5 kg/h, 500 kg of titanium-group metal micro-dispersion water can be obtained. Produced water dispersant is retrieved from the outlet and fed to a filter system. The filter system comprises filter membranes of 50 microns, 25 microns, 3 microns, 0.5 micron and 0.1 micron, and by passing the produced water through these filter membranes sequentially a titanium micro-dispersion water containing super-fine titanium grains and a very small amount of molten titanium is obtained. At this time, a part of titanium takes on a crystalline structure, to which titanium atoms will rearrange themselves to form grains of near-spherical shape, thus attaining an energetically stable state.

A pressure-resistant container that can withstand the pressures generated during underwater plasma discharge is sufficient for use as the water tank (3). The proposed production method is very efficient, because the apparatus can produce a water solution of super-fine titanium grains almost successively through pulsed plasma discharge and the apparatus itself can be constructed in a small size. Since the construction is simple and does not require an overly high pressure resistance, the apparatus also ensures excellent safety.

In the above example, titanium was used as material titanium-group metal. A separate experiment involving zirconium also produced a similar micro-dispersion water.

### Trial Test of Hair-Repair Liquid Made of Titanium Micro-Dispersion Water

Ten male and female subjects were instructed to use as a hair-repair liquid the titanium micro-dispersion water obtained in the above example in which super-fine titanium grains are micro-dispersed, and the effects and efficacies of the treatment were verified under different conditions, including washing hair with the liquid and spraying/applying it to hair.

### Use Conditions and Test Results

Use conditions: Washing hair in a basin (3 liters per wash)
   : Spraying onto hair with a spray (10 cc per application)
   : Applying onto hair using a brush (10 cc per application)

1.10 male and female subjects (The subjects used the liquid at varying frequencies)
2. Efficacies

| | |
|---|---|
| Hair became less tangling | 5 persons |
| Fallen hair became less | 8 persons |
| Hair became shiner | 7 persons |
| Hair became darker | 1 person |
| Hair became denser | 6 persons |
| Scalp became smoother | 7 persons |
| Dandruff became less | 7 persons |

As evident from the above test results, many of those who washed their hair with the titanium micro-dispersion water obtained by the present invention or applied/sprayed it to their hair cited recovery of damaged hair, improved hair condition, smoother scalp, reduced dandruff and other improvements. Therefore, it is found that a hair-repair liquid comprising the titanium micro-dispersion water obtained by the present invention has remarkable medical and/or cosmetic effects.

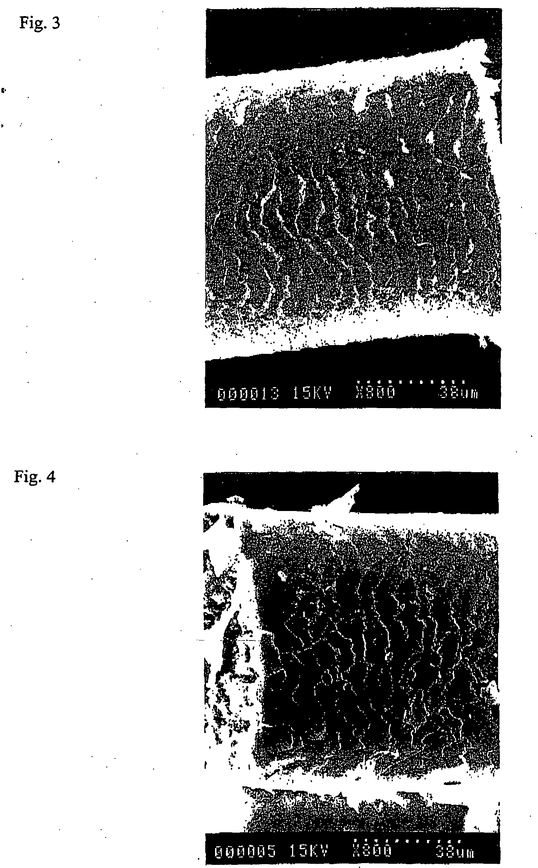

Fig. 3 shows a microphotograph of hair treated with the hair-repair liquid made of titanium micro-dispersion water used in the above test. For comparison, Fig. 4 gives a microphotograph of hair treated with normal water.

When the microphotographs in Fig. 3 and 4 are compared, it is evident that the hair treated with the titanium micro-dispersion water obtained by the present invention is less fuzzy and rough on the surface.

### Industrial Field of Application

The present invention, whose structure is explained above, provides a hair-repair liquid comprising newly developed titanium-group metal micro-dispersion water, as well as its production method and apparatus, and allows for efficient production of a hair-repair liquid made of bioactive micro-dispersion water of titanium-group metal containing super-fine titanium-group metal grains in micro-dispersed state. A number of trial tests also found that the hair-repair liquid comprising the obtained titanium-group metal micro-dispersion water is also useful as healthy drinking water, health supplements, cosmetics, food preservatives, freshness-keeping agents for food, insect repellents and deodorants, which suggests beneficial effects of such water in industrial applications.

## Claims

1. A hair-repair liquid comprising a micro-dispersion water of titanium-group element in which super-fine titanium-group metal grains are micro-dispersed.

2. A method for producing a hair-repair liquid in which super-fine titanium-group metal grains are micro-dispersed, wherein a high-voltage discharge generator equipped with a titanium-group metal electrode and a counter electrode is used to cause plasma discharge between the electrodes in water, thereby allowing super-fine titanium-group metal grains to micro-disperse in water.

3. An apparatus for producing a hair-repair liquid in which super-fine titanium-group metal grains are micro-dispersed, which comprises a power supply for high-voltage/current discharge, a high-voltage discharge generator equipped with a titanium-group metal electrode and a counter electrode, a water tank, a water inlet to the water tank, an outlet for generated water solution of super-fine titanium-group metal grains, and a discharge pump.
